# EUROPEAN PATENT APPLICATION

(11) **EP 2 045 806 A2**
(43) Date of publication of application: **08.04.2009**
(21) Application number: 07291189.4
(22) Date of filing: 01.10.2007
(51) Int. Cl.: G11B 7/249, G11B 7/246, C09B 69/04, G11B 7/247, C09B 67/00

(54) **Use of indolinium diazamethine cations for optical data recording**

(71) Applicant: CLARIANT INTERNATIONAL LTD, 4132 Muttenz 1 (CH)
(72) Inventor: Klein, Cédric, 67170 Brumath (FR); Graciet, Jean-Christophe, 68128 Village-Neuf (FR); Winter, Martin, Alexander, 79400 Kandern (DE)

(57) **Abstract**

The present invention relates to specific pentamethine cyanine azo complex dye compounds and their use as dyes in optical layers for optical data recording, preferably for optical data recording using a laser with a wavelength from 630 to 670 nm.

The invention further relates to an optical layer comprising said dyes and to a write only read many (WORM) type optical recording medium capable of recording and reproducing information with radiation of a red laser, which employs said pentamethine cyanine azo complex type dye in the optical layer.

## Description

The present invention relates to specific pentamethine cyanine azo complex dye compounds and their use as dyes in optical layers for optical data recording, preferably for optical data recording using a laser with a wavelength from 630 to 670 nm.

The invention further relates to an optical layer comprising said dyes and to a write only read many (WORM) type optical recording medium capable of recording and reproducing information with radiation of a red laser, which employs said pentamethine cyanine azo complex type dye in the optical layer.

Recently, organic dyes have attracted considerable attention in the field of diode-laser optical storage. Commercial recordable compact discs (CD-R) and recordable digital versatile discs (DVD-R) can contain, as recording layer, numerous dyes based on phthalocyanine, hemicyanine, cyanine and metallized azo structures. These dyes are suitable in their respective fields with the laser wavelength criteria. Other general requirements for dye media are strong absorption, high reflectance, high recording sensitivity, low thermal conductivity as well as light and thermal stability, durability for storage and non-toxicity.

For industrial application, organic dyes in the field of diode-laser optical storage have to be suitable for the spin coating process to prepare thin films, i.e. they have to be sufficiently soluble in the organic solvents generally applied in the spin coating process.

Write only read many (WORM) type and erasable type optical recording media reproduce information by detecting variations in the reflectivity caused by physical deformation, by alterations of optical characteristics as well as by phase properties and magnetic properties of a recording layer before and after the recording.

Recordable compact discs with a storage capabilities up to 680 MBytes (CD-R) are an example of a WORM type optical recording medium. Recently, digital versatile discs (DVD) with increased information storage capabilities up to 4.7 GBytes and the respective DVD-R have been commercialized.

The DVD-R technology currently adopts as a light source a red diode laser with a wavelength of 630-670 nm. Thereby the pit size and track interval can be reduced, increasing the information storage capacity by up to 6-8 times compared to CD-R's.

Blu-ray^{®} discs (Blu-ray^{®} disc is a standard developed by Hitachi Ltd., LG Electronics Inc., Matsushita Electric Industrial Co. Ltd., Pioneer Corporation, Royal Philips Electronics, Samsung Electronics Co. Ltd., Sharp Corporation, Sony Corporation, Thomson Multimedia) are going to be the next milestone in optical recording technology. Its new specification increases the data storage up to 27 GBytes per recording layer for a 12 cm diameter disc. By adopting a blue diode laser with a wavelength of 405 nm (e.g. by use of GaN or SHG laser diodes), the pit size and track interval can be further reduced, again increasing the storage capacity by an order of magnitude.

The construction of optical data recording media is known in the art. An optical data recording media generally comprises a substrate and a recording layer, the optical layer. Usually discs or wavers of organic polymeric materials are used as substrates. Preferred substrates are polycarbonate (PC) or polymethylmethacrylate (PMMA). The substrate has to provide an even and uniform surface of high optical quality. The optical layer is deposited thereon in a thin and uniform film of high optical quality and defined thickness. Finally, a reflective layer, e.g. silver, gold or copper, is deposited upon the optical layer.

Advanced optical data recording media may comprise further layers, such as protective layers, adhesive layers or additional optical layers.

To provide for a thin and uniform film of the optical layer, the material is usually deposited by spin coating, vacuum evaporation, jet coating, rolling coating, or soaking. The preferred process in industry is spin coating to form an optical layer of about 70 nm to 250 nm thickness. For the application in the spin coating process, the material of the optical layer has to be highly soluble in organic solvents.

For these application in the field of optical data recording, high lightfastness, high sensitivity, high readout stability, good solubility in the solvents used in the application, suitable temperature stability and suitable decomposition temperature among other parameters is required.

EP 1 347 030 A discloses Trimethine cyanine dyes and their use as light absorbents.

There was a need for dye compounds for optical layers in optical data recording media, in particular for DVD-R optical data recording media, which meet todays technical requirements.

Surprisingly it has been found that compounds comprising certain cationic pentamethine cyanine derivatives and certain anionic azo metal complexes are useful as dye compounds in optical layers for optical data recording media, in particular for DVD-R optical data recording media.

In the following text, "halogen" represents F, Cl, Br or I, preferably F, Cl or Br, more preferably F or Cl, even more preferably Cl, if not otherwise stated; "halide" represents F-, Cl-, Br- or I-, preferably Cl- or I-, if not otherwise stated; "alkyl" represents linear and branched alkyl; and "alkoxy" represents linear and branched alkoxy; any alkyl and cycloalkyl groups being unsubstituted, partially or completely substituted by halogen; if not otherwise stated.

Subject of the invention is a compound of formula (I),

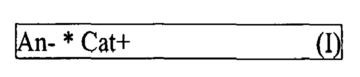

Cat+ being a compound of formula (II);

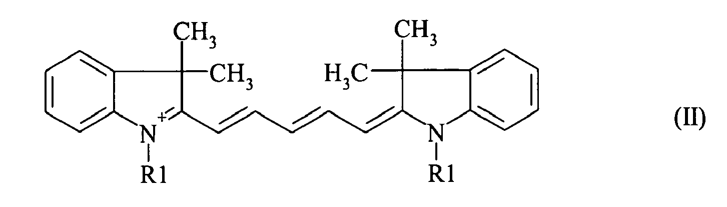

An- being a compound of formula (III); 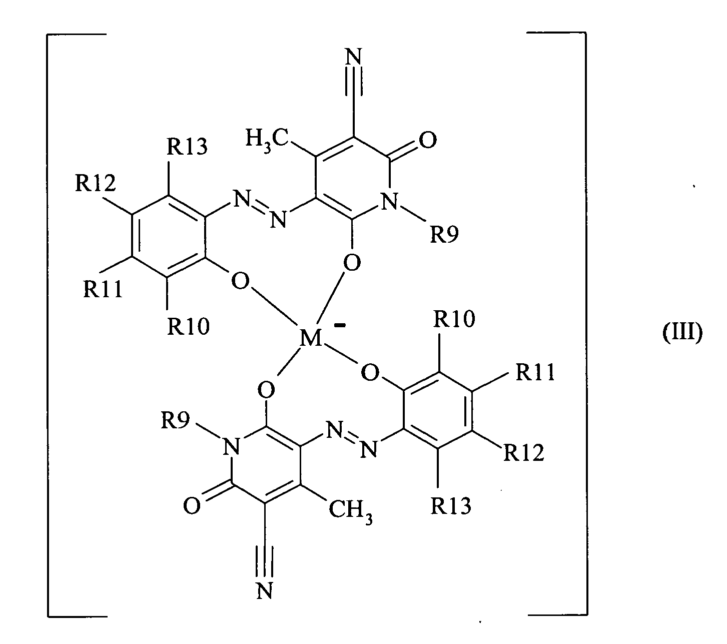
- M: represents a trivalent metal atom, preferably selected from groups 3, 4 5, 6, 7, 8, 9, 10, 11 and 12 of the Periodic Table of the Chemical Elements;
- R9: is n-butyl, allyl or propargyl;
- R1: represents C₁₋₁₀ alkyl;
- R10, R11, R12 and R13: are identical or different and independently from each other selected from the group consisting of H, CN, CF₃, halogen, NO₂, OH, SH, SO₂-NR²¹R²², CO-R²⁰, SO₂R²⁰, CO-NR²¹R²², C₁₋₁₀ alkyl, C₃₋₁₀ cycloalkyl, the C₁₋₁₀ alkyl and the C₃₋₁₀ cycloalkyl being independently from each other unsubstituted or substituted by 1 to 4
identical or different substituents, the substituents being independently from each other selected from the group consisting of C₁₋₁₀ alkyl, halogen, OH, CN, CF₃, C₆₋₁₂ aryl and NR²¹R²², C₆-C₁₂ aryl, O-C₆₋₁₂ aryl, S-C₆₋₁₂ aryl, the C₆₋₁₂ aryl and the O-C₆₋₁₂ aryl and the S-C₆₋₁₂ aryl being unsubstituted or substituted by 1 to 4 identical or different substituents, the substituents being independently from each other selected from the group consisting of C₁₋₁₀ alkyl, C₃₋₁₀ cycloalkyl, OH, NO₂, CN, halogen, CF₃, C₆₋₁₂ aryl, O-C₁₋₁₀ alkyl, S-C₁₋₁₀ alkyl and NR²¹R²², O-C₁₋₁₀ alkyl, S-C₁₋₁₀ alkyl, O-C₃₋₁₀ cycloalkyl, S-C₃₋₁₀ cycloalkyl, NHCOR²⁰ and NR²¹R²²;
with the proviso, that if R9 is n-butyl, then R10 is -NHCOCH₃;
the R²¹ and R²² residues are identical or different and independently from each other selected from the group consisting of H, C₁₋₁₀ alkyl, C₆₋₁₂ aryl and C₁₋₁₂ alkyl-NR²³R²⁴;
the R²³ and R²⁴ residues are identical or different and independently from each other selected from the group consisting of H, C₁₋₁₀ alkyl and C₆₋₁₂ aryl;
the R²⁰ residues are identical or different and independently from each other selected from the group consisting of OH, C₁₋₆ alkyl, C₆₋₁₀ aryl and O-C₁₋₆ alkyl.

Preferably,
- M: is selected from the group consisting of Co, Cr, Fe and Al;
- R9: is n-butyl, allyl or propargyl;
- R1: represents C₁₋₄ alkyl;
- R10: is H or -NHCOCH₃;
with the proviso, that if R9 is n-butyl, then R10 is -NHCOCH₃;
- R11: is H or NO₂;
- R12: is NO₂;
- R13: is H.

More preferably,
- M: is selected from the group consisting of Co, Fe and A1, more preferably Co;
- R9: is n-butyl, allyl or propargyl;
- R1: represents n-butyl;
- R10: is H or -NHCOCH₃;
with the proviso, that if R9 is n-butyl, then R10 is -NHCOCH₃;
- R12: is NO₂;
- R11 and R13: are H.

Especially,
Cat+ is a compound of formula (1); 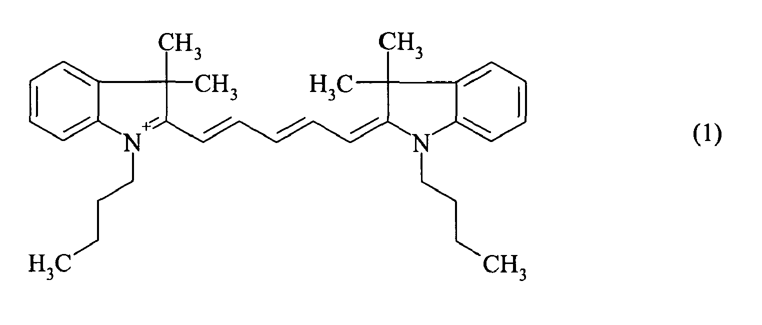 and

An- is a compound of formula (10), (11) or (12).

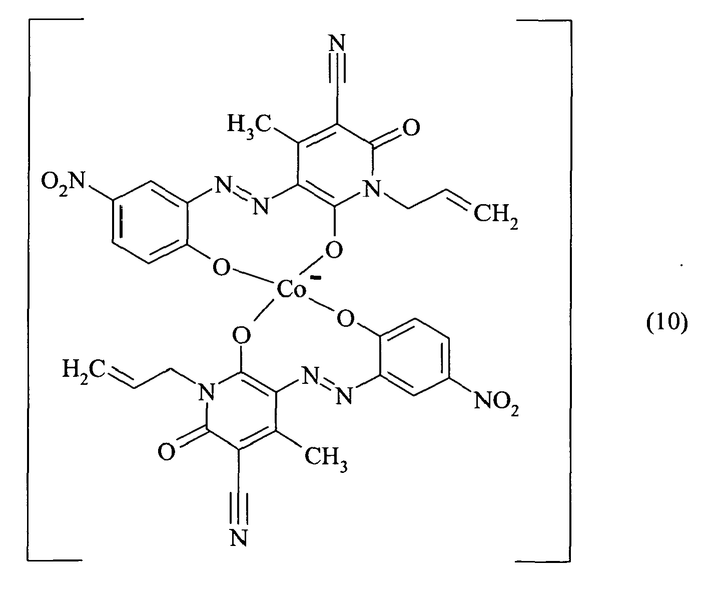

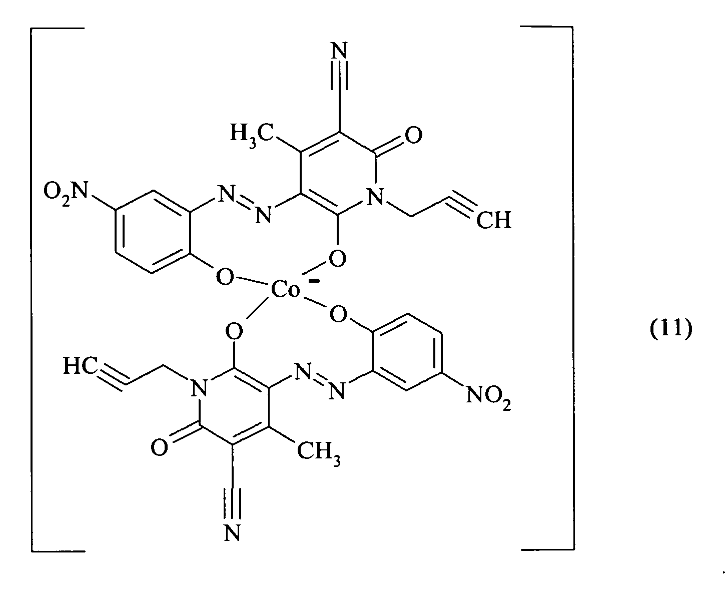

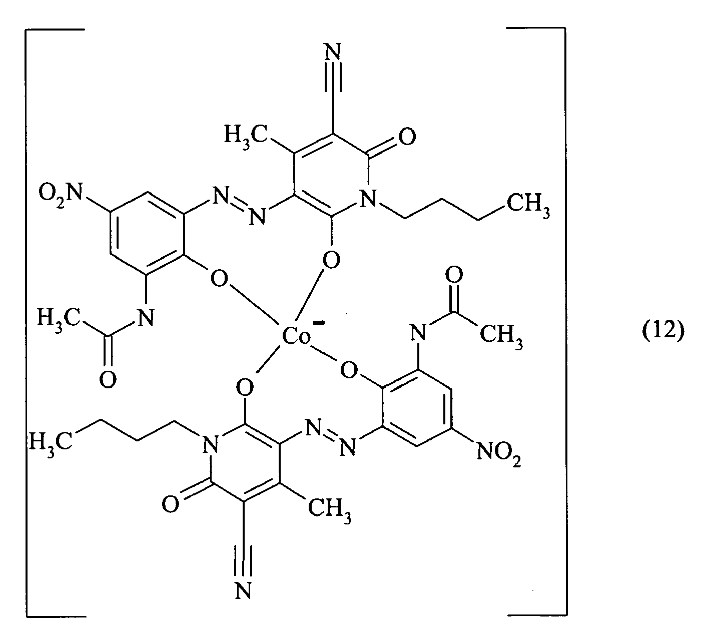

More especially, the compounds of formula (I) are selected from the group consisting of compounds of formulae (10_1), (11_2) and (12_1).

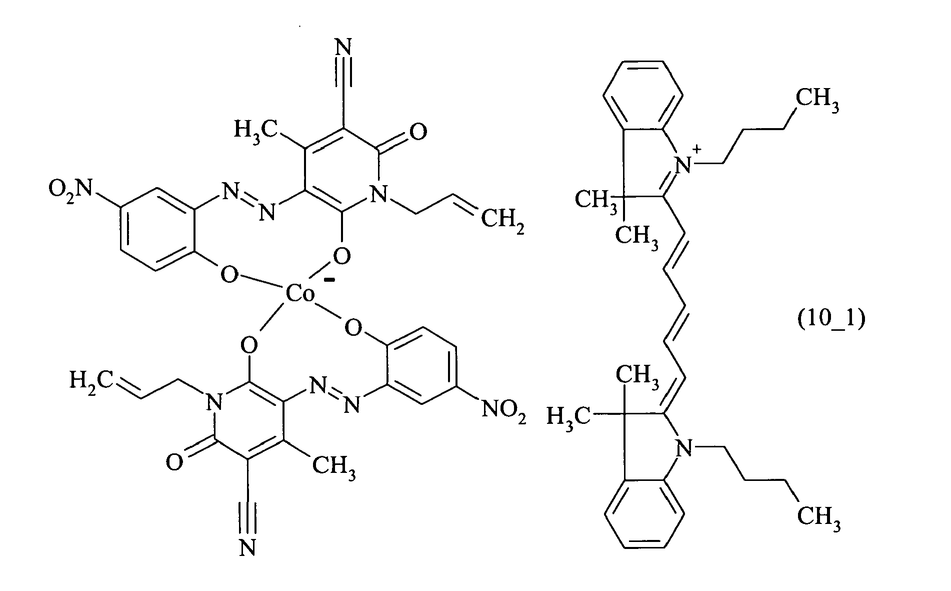

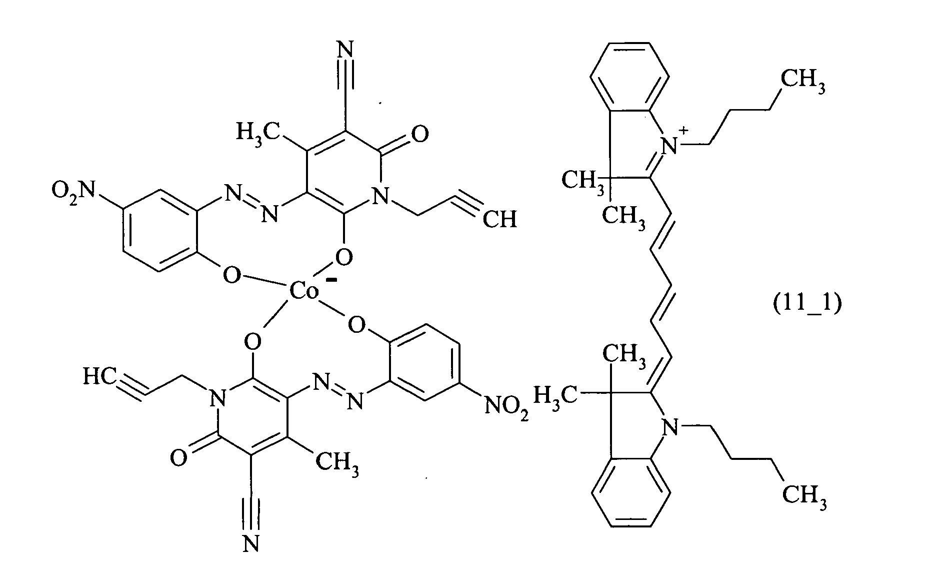

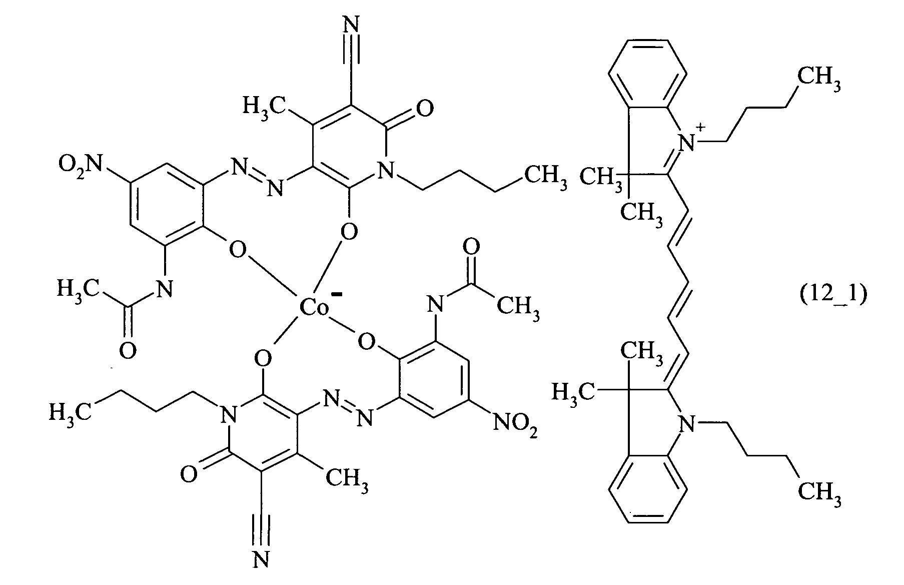

### Composition C

A further subject of the invention is a composition C comprising a component A and a component B,
the component A being a compound of formula (I) as defined above, also with all its preferred embodiments as described above, and
the component B being a compound of formula (10_20); 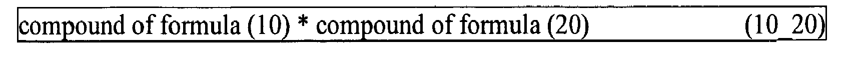 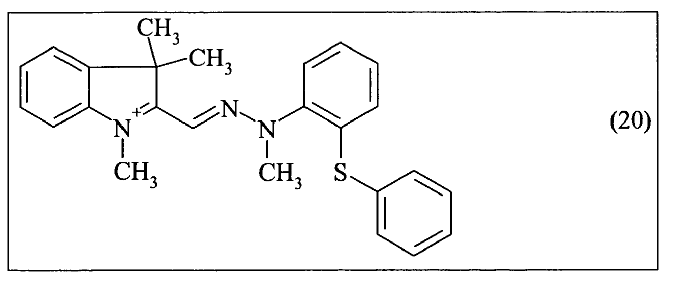 with the compound of formula (10) being as defined above.

Component A comprises at least one, preferably 1, 2 or 3, more preferably of 1 or 2 compounds of formula (I).

Preferably component A is selected from the group consisting of compound of formula (10_1), (11_1) and (12_1).

More preferably, component A is the compound of formula (11_1), or the compound of formula (12_1), or component A consists of the compound of formula (11_1) and the compound of formula (12_1).

Preferably, the ratio of the weight of component A to the weight of component B in the composition C is of from between 1 to 99 and 99 to 1, more preferably of from between 10 to 90 and 90 to 10, even more preferably of from between 20 to 80 and 80 to 20, especially of from between 50 to 50 and 80 to 20.

Preferably, the composition C comprises preferably 10 to 100 % by weight, more preferably 25 to 100 % by weight, even more preferably 50 to 100% by weight, especially 75 to 100 % by weight, in particular 90 to 100 % by weight, based on the weight of the total composition C, of the combined amounts of the component A and component B.

Preferably, further components, which are present in the compositions C, are the "further customary components" mentioned below in the section "(d) Optical layer".

Further subject of the invention is the use of a compound of formula (I), also in all the preferred embodiments of formula (I) as described above, or of a composition C, also in all the preferred embodiments of the composition C as described above, in an optical layer, preferably in an optical layer for optical data recording, more preferably the use of a compound of formula (I) or of a composition C as a dye in an optical layer for optical data recording.

Further subject of the invention is the use of a compound of formula (I), also in all the preferred embodiments of formula (I) as described above, or of a composition C, also in all the preferred embodiments of the composition C as described above, as dye compounds in DVD-R optical data recording media.

Further subject of the invention is the use of a compound of formula (I), also in all the preferred embodiments of formula (I) as described above, or of a composition C, also in all the preferred embodiments of the composition C as described above, in a write only read many (WORM) type optical data recording medium capable of recording and reproducing information with radiation of a laser, preferably of a red laser at a wavelength from 630 to 670 nm, more preferably at around 650 nm, which comprises an optical layer comprising at least one compound of formula (I).

### Preparation of the Composition C

A further subject of the invention is the use of a compound of formula (10_20) for the preparation of a composition C.

A further subject of the invention is the use of a compound of formula (I) as defined above, also in all it's preferred embodiments, for the preparation of a composition C.

A further subject of the invention is a process for the preparation of the composition (C) comprising the components (A) and (B), by physically combining the (individual) components (A) and (B).

The composition (C) is prepared by physically combining the (individual) components (A) and (B), preferably by blending or mixing, the blending or mixing is preferably done in the solid, molten or dissolved state of the components; preferably the blending or mixing is done by dry blending, by mixing the slurries or the solutions of the components; or the mixing is done by a combination of any of the mentioned measures.

For the dry blending, the component (A) is mixed physically with the component (B) in a mill, in a shaker or in any other mechanical device leading to homogeneous mixture of components (A) and (B).

For the slurry blending, also called wet mixing, the component (A) is mixed physically with the component (B) in a solvent. The solvent is preferably at least one organic solvent, water or a mixture of at least one organic solvent with water.

In the case that the components are blended in form of their solutions, the components are dissolved in the solvent either individually or in form of a premixture of the components.

More preferably, the solvent is selected from the group consisting of alcohols, chlorinated solvents and ketones, even more preferably of methanol, ethanol, propanol, butanol, tetrafluoropropanol, dichloromethane, chloroform, acetone, methyl ethyl ketone and methyl tert-butyl ketone.

To obtain dry blends from the slurry or the solution, the solvent is removed by conventional methods known in the art, preferably by filtration or distillation, and the resulting presscake is dried.

### Preparation of the Compounds of Formula (I) and (10_20)

A further subject of the invention is a process for the preparation of a compound of formula (I), also in all their preferred embodiments of the formula (I) as described above, especially of a compound of formulae (10_1), (11_1) or (12_1), by metathesis reaction between a compound of formula (III_6), 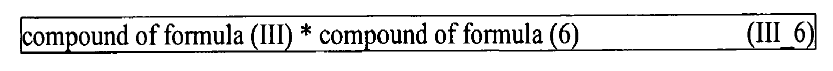 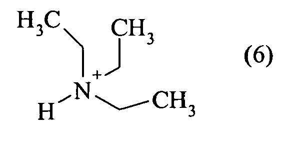 the compound of formula (III) also being in all the preferred aspects of the formula (III) as described above,
and the a compound of formula (II_salts);

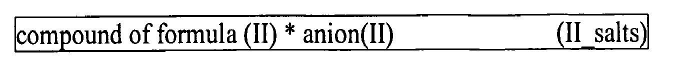

the compound of formula (II) also being in all the preferred aspects of the formula (II) as described above,
the anion(II) being selected from the group consisting of halides, preferably chloride, bromide and iodide, even more preferably being iodide.

Preferably, the compound of formula (II_salts) is a compound of formula (1_I).

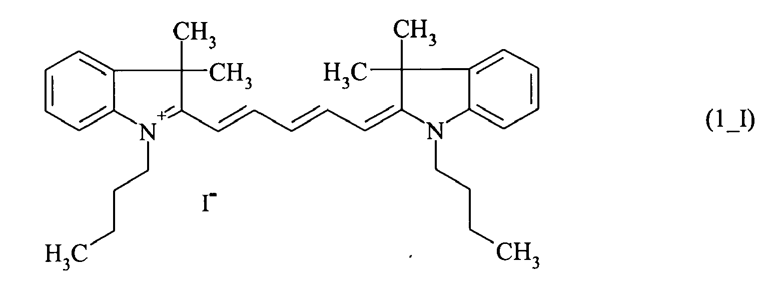

Metathesis reaction within the meaning of the invention signifies an exchange of ions between different salts.

The metathesis reaction is done preferably by mixing the respective compounds of formula (III_6) with the respective compounds of formula (II_salts).

The metathesis reaction can be carried out in suspension or in solution, preferably it is carried out in suspension.

The solvents that can be used for the metathesis reaction are water, solvents and mixtures thereof. The solvents are preferably selected from the group consisting of C₁₋₈ alcohols, nitriles, preferably acetonitrile, acetone, aromatic solvents such as toluene or chlorobenzene, DMF, DMSO, NMP.

More preferred solvents are C₁₋₈ alcohols and nitrile, especially ethanol and acetonitrile.

The metathesis reaction is generally done at temperatures between 20°C to 200°C, preferably at temperatures between 50°C to 170°C, particularly preferably at temperatures between 60°C to 150°C, further particularly preferably the metathesis reaction is carried out at reflux temperature under atmospheric pressure.

The metathesis reaction is preferably done under atmospheric pressure.

Particularly, the metathesis reaction is carried out under reflux at the reflux temperature of the solvent system used at atmospheric pressure.

Preferably, the reaction time for the metathesis reaction is preferably of from 30 min to 30 hours, more preferably of from 1 hour to 24 hours.

Preferably the compounds of formula (I) are isolated following standard methods, usually they form a precipitate, which is preferably isolated by filtration and dried.

When the compounds of formula (I) are prepared, and also depending on the molar ratio between the compound of formula (III_6) and the compound of formula (II_salts), the cation of the compound of formula (III_6) may not be exchanged completely against the cation of the compound of formula (II_salts), resulting in a mixture of compounds comprising a compound of formula (III_6), a compound of formula (I) and possibly a compound of formula (II_salts).

Preferably, compounds of formulae (10_6), (11_6) or (12_6), as specified in table (AA), were used for the metathesis reaction.

| **Table (AA)** | | |
|---|---|---|
| **Compounds of formula (III_6): Compound of formula** | **An-: compound of formula** | **Cat+: compound of formula** |
| (10_6) | (10) | (6) |
| (11 6) | (11) | (6) |
| (12-6) | (12) | (6) |

Another subject of the invention is the use of a compound of formula (III_6), also in all it's preferred embodiments as described above, especially the use of a compound of formula (10_6), (11_6) or (12_6), for the preparation of a compound of formula (I). Another subject of the invention is a compound of formula (II_salts), also in all it's preferred embodiments as described above, especially a compound of formula (1_I).

Another subject of the invention is the use of a compound of formula (II_salts), also in all it's preferred embodiments as described above, especially the use of a compound of formula (1_I), for the preparation of a compound of formula (I).

The compound of formula (10_20) can be prepared in analogy to the procedure for the preparation of the compounds of formula (I), by using the respective precursor, i.e. a compound of formula (20_salts), instead of a compound of formula (II_salts), and a compound of formula (10_6), 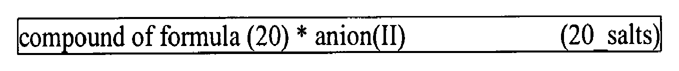 with the anion(II) being as defined above.

### Preparation of An-

The compound of formula (III_6), also in all the preferred embodiments of the formula (III_6) as described above, especially a compound of formulae (10_6), (11_6) or (12_6) as defined in table (AA), is prepared
by a complexing reaction of a respective metal salt with the respective compound of formula (d), in the presence of triethylamine, 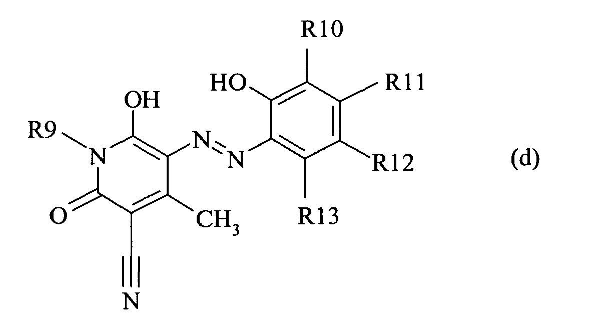 with R9, R10, R11, R12 and R13 being as defined in formula (III), also in all their preferred embodiments as described above.

Particularly the compounds of formulae (10_6), (11_6) or (12_6) are prepared by a complexing reaction of a respective metal salt with the respective compounds of formulae (d1), (d2) or (d3), also called azo ligands, in the presence of triethylamine with in situ formation of compound of formula (6) by protonation.

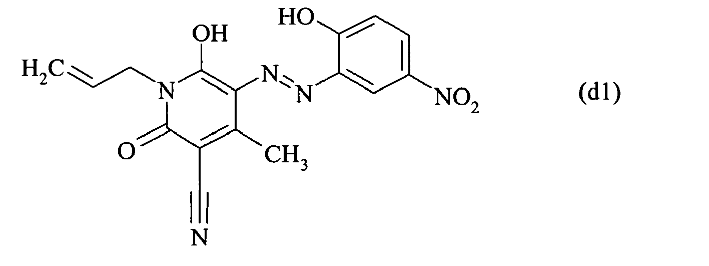

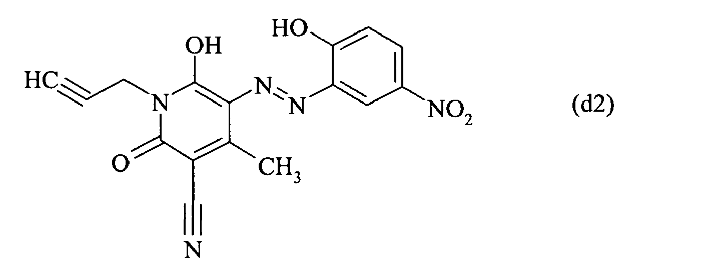

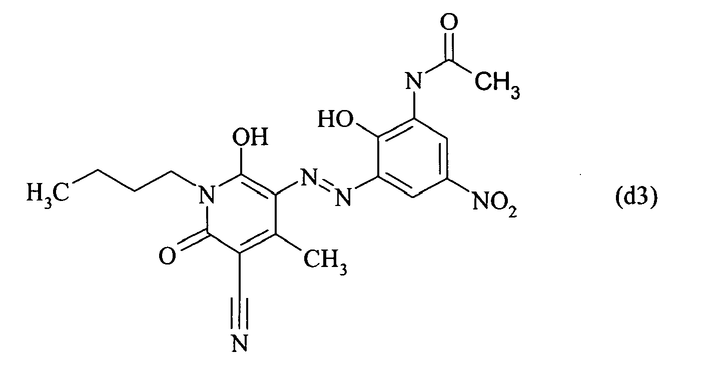

The complexing reaction is preferably done using the required stoichiometric ratios between the azo ligands and the metal salt; each of the reactants may be used in excess with respect to the other reactant, preferably one equivalent of a metal salt and two equivalents of the combined amounts of one or two, preferably of one azo ligand, are used.

Preferably, the complexing reaction is done with a solution of one equivalent of a metal salt and with a boiling solution of two equivalents of the respective azo ligands.

Preferably, the complexing reaction is done with a trivalent metal salt, more preferably Co, Fe or Al. In another preferred embodiment, the complexing reaction is done with a divalent metal salt, more preferably Co or Fe, under aerobic conditions. More preferably, particularly in case of the compounds of formulae (10_6), (11_6) or (12_6), the metal of the metal salt is derived from a divalent metal, and the complexing reaction is carried out in the presence of preferably 1 to 4, more preferably 2.5 to 4, even more preferably 2.9 to 3.2, especially 3 equivalents of triethylamine for each equivalent of the combined amounts of the ligands under aerobic conditions.

The aerobic condition ensures, that the divalent metal atom is converted during the complexing reaction to a trivalent metal atom, and that the trivalent metal atom is incorporated into its four-fold coordination in the complex, resulting in an anionic charge on the final complex.

The azo ligand can be added to the metal salt or vice versa.

Preferably, compound of formula (6) is formed during the complexing reaction, more preferably compound of formula (6) is formed during the complexing reaction when a metal salt derived from a divalent metal is used in the presence of triethylamine under aerobic conditions in the complexing reaction, with the metal salt being especially preferably CoSO₄*7H₂O or FeSO₄*7H₂O.

The complexing reaction can be carried out in suspension or in solution, preferably in solution.

The solvent preferably used in the complexing reaction is water, a non-aqueous solvent or a mixture thereof. The non-aqueous solvent is preferably selected from the group consisting of C₁₋₈ alcohols, nitriles, preferably acetonitrile, ketones, preferably acetone, aromatic solvents, preferably toluene or chlorobenzene, and dipolar aprotic solvents, preferably DMF, DMSO, NMP, pyridine and mixtures thereof.

More preferred solvents are C₁₋₈ alcohols, especially ethanol, acetonitrile and pyridine.

The solvent used for the complexing reaction can be different from the solvent used for the metathesis reaction.

It is also possible to add the metal salt already at an earlier stage of the synthesis of the azo ligand or their precursors, preferably before, during or after the azo coupling reaction, more preferably after the azo coupling reaction to the resulting suspension or solution of the azo ligand.

The complexing reaction and the metathesis reaction can be carried out separately in two steps, or jointly in one step.

Even more preferably, the compounds of formula (III_6) are isolated after synthesis, and the metathesis reaction is carried out in a separate step.

The complexing reaction is preferably done at a temperature of from 0°C to 200°C, more preferably of from 5°C to 170°C, even more preferably of from 20°C to 150°C, particularly of from 60°C to 150°C.

The complexing reaction is preferably done under atmospheric pressure.

Particularly, the complexing reaction is carried out under reflux at the reflux temperature of the solvent system used at atmospheric pressure.

Preferably, the reaction time for the complexing reaction is preferably of from 30 min to 30 hours, more preferably of from 1 hour to 24 hours.

Preferably the compound of formula (III_6) is isolated following standard methods, usually the compound of formula (III_6) forms a precipitate which is isolated, preferably by filtration, and preferably followed by drying.

The metal salt is a derived from a divalent or a trivalent metal with the metal selected preferably from the group consisting of Co, Al, Fe and Cr. The salts of these metals are preferably sulfates, halides (preferably fluoride, chloride, bromide, iodide, more preferably chloride and bromide, especially chlorides) and salts of organic acids, preferably acetates, and their respective hydrates.

In case of a divalent metal the metal has to be converted to its trivalent form. Preferably this is done during complexing reaction at presence of triethylamine under aerobic conditions.

Preferred metal salts are derived from Co, Fe and Al. More preferred metal salts are for example cobalt-, iron- or aluminium-halides, more preferable chlorides, cobalt-, iron- or aluminium-sulfates; cobalt- or aluminium-acetates, and their respective hydrates, especially preferably AlCl₃, Al₂(SO₄)₃, Al₂(SO₄)₃*18 H₂O, CoSO₄*7 H₂O and FeSO₄* 7H₂O, FeCl₃*H₂O, FeCl₃*6H₂O, Co(acetylacetonate)₃, Fe(acetylacetonate)₃, Fe₂(SO₄)₃*xH₂O or iron salts of organic acids, preferable formate, gluconate, citrate and oxalate.

More preferably the complexing reaction is done with a metal salt derived from a divalent metal under aerobic conditions, with the metal salt preferably being CoSO₄*7H₂O or FeSO₄*7H₂O, preferably in the presence of triethylamine.

The compounds of formula (d), especially the compounds of formula (d1), (d2) and (d3), are preferably prepared by an azo coupling reaction of the respective compound of formula (c), especially the compounds of formula (c1), (c2) and (c3), also called coupling agent, with the respective compound of formula (b), also called diazo component; the compound of formula (b) being preferably prepared by diazotization reaction of the respective compound of formula (a), also called amine compound; 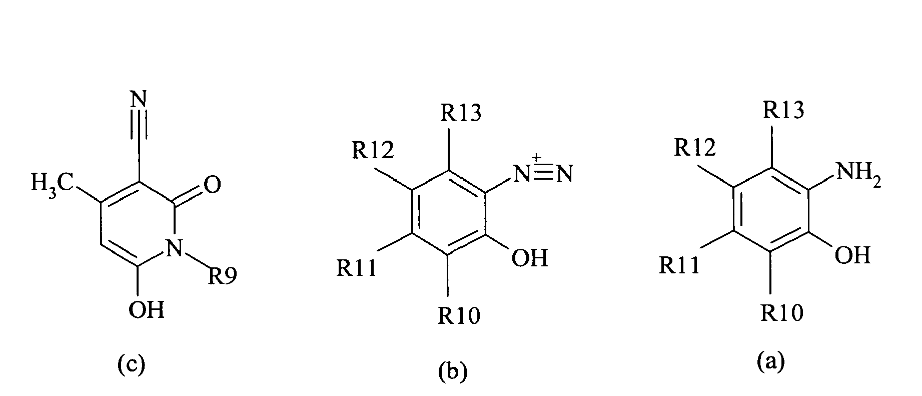 wherein R9, R10, R11, R12 and R13 have the same meaning as described above, also with all their preferred embodiments.

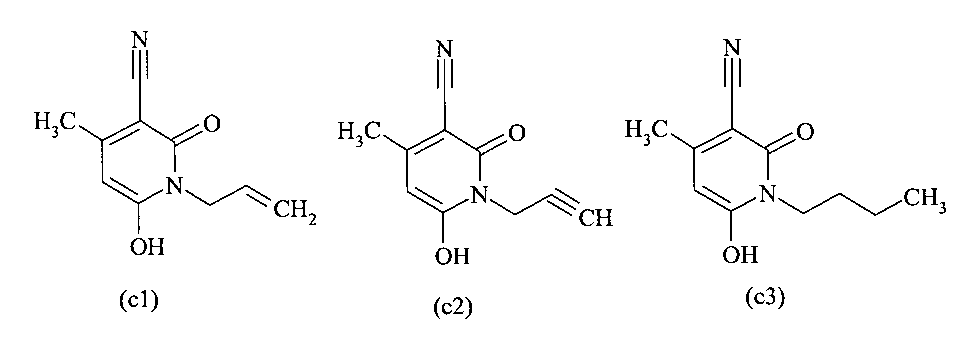

The diazo component has preferably chloride Cl- as counter ion, since the diazotization reaction of the amine compound preferably is done in aqueous hydrochloride acid.

The amine compounds are known substances and can be prepared according to or in analogy to known procedures.

It is possible to use more than one amine component and/or more than one coupling agent resulting in the respective mixture of the azo ligands.

The azo coupling reaction is preferably carried out in suspension or in solution.

The azo coupling reaction is preferably carried out in water, non-aqueous solvents and in mixtures thereof. Non-aqueous solvents are preferably selected from the group consisting of alcohols, more preferably methanol, ethanol, propanol, butanol, pentanol, dipolar aprotic solvents, preferably dimethylformamide (DMF), DMSO, dimethylacetamide or N-methyl-pyrrolidinone (NMP) and pyridine, and water-immiscible solvents, preferably toluene or chlorobenzene. More preferably the azo coupling reaction is carried out in water.

The azo coupling reaction is preferably carried out with a stoichiometric ratio between the coupling component and the diazo component.

The azo coupling reaction is generally done at a temperature of from -30°C to 100°C, preference being given to temperatures of-10°C to 30°C, and particular preference to temperatures of-5°C to 30°C.

The azo coupling reaction may be carried out in an acidic as well as in an alkaline medium. Preference is given to pH < 10, particular preference to pH 3 to 9.

Preferably, the reaction time for the azo coupling reaction is preferably of from 30 min to 30 hours, more preferably of from 1 hour to 24 hours.

Preferably, the azo coupling reaction is done under atmospheric pressure.

Preferably the azo ligand is isolated following standard methods, in case of a precipitate preferably by filtration followed preferably by washing and drying.

A further subject of the invention is a process for the preparation of the compound of formula (c), i.e. the coupling agent, by a condensation reaction of an intermediate amide, i.e. of a compound of formula (c_amide), with a compound of formula (c_aaester), preferably under basic conditions; and a further subject of the invention is a process for the preparation of the intermediate amide, i.e. the compound of formula (c_amide), by a condensation reaction of a respective amine compound, i.e. of a compound of formula (c_amine), with cyanoacetic acid ethylester, i.e. compound of formula (c_cyanoaaester); R9 in the formulae having the same meaning as described above, also with all its preferred embodiments.

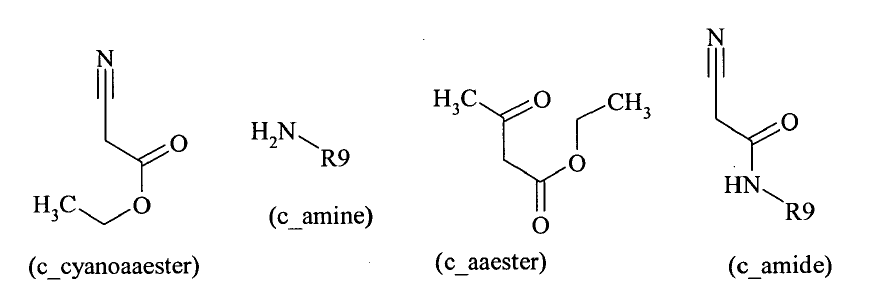

The compound of formula (c_amine) is a known substance and can be prepared according to or in analogy to known procedures.

The intermediate amide, i.e. the compound of formula (c_amide), can be isolated after precipitation or by distillation, preferably it is not isolated and the two steps are carried out without isolation of the intermediate amide.

Each condensation reaction resulting in the compound of formula (c) or in the compound of formula (c_amide) respectively, is preferably carried out in non-aqueous solvents and in mixtures thereof. Non-aqueous solvents are preferably selected from the group consisting of alcohols, more preferably methanol, ethanol; propanol, butanol, pentanol, further dipolar aprotic solvents, preferably dimethylformamide (DMF), DMSO, dimethylacetamide or N-methyl-pyrrolidinone (NMP) and pyridine, and further water-immiscible solvents, preferably toluene, chlorobenzene, hexane, cyclohexane or heptane. More preferably the condensation reaction is carried out in toluene or ethanol.

Each condensation reaction is preferably carried out with a stoichiometric ratio between the compound of formula (c_aaester) and the compound of formula (c_amine); and between the compound of formula (c_amine) and the compound of formula (c_cyanoaaester) respectively.

Each condensation reaction is preferably done at a temperature of from 0°C to 200°C, more preferably of from 10°C to 180°C, even more preferably of from 25°C to 150°C.

Preferably, water and/or ethanol formed during the condensation reaction is distilled off during the reaction.

The reaction time for each condensation reaction is preferably of from 30 min to 30 hours, more preferably of from 1 hour to 24 hours.

Preferably, each condensation reaction is done under atmospheric pressure.

The condensation reaction for the preparation of the compound of formula (c), i.e. for the coupling agent, is preferably carried out in the presence of a organic or inorganic base as catalyst, preferably selected from the group consisting of alkaline hydroxides, preferably NaOH and KOH, further organic aromatic amines, preferably pyridine, further organic alkylamines, preferably triethylamine, piperidine and lutidine, further sodium- or potassium alcoholates, preferably sodium methoxide or sodium ethoxide, and further basic ion exchange resins.

Preferably the coupling agent is isolated following standard methods, in case of a precipitate preferably by filtration followed preferably by washing and drying, in case of a solution, the solution is preferably concentrated until precipitation, preferably by distillation.

### Preparation of Cat+

Another subject of the invention is the preparation of a compound of formula (II_salts), especially of the compound of formula (1_I), by a condensation reaction between compound of formula (IIc_salt) and compound of formula (IId), wherein R1 has the same meaning as described above, also with all its preferred embodiments; and the anion(II) being as defined above with all its preferred embodiments, more preferably anion(II) in chloride or iodide, even more preferably, in case of the compound of formula (IIc_salt), anion(II) is iodide, and in case of the compound of formula (IId), anion(II) is chloride.

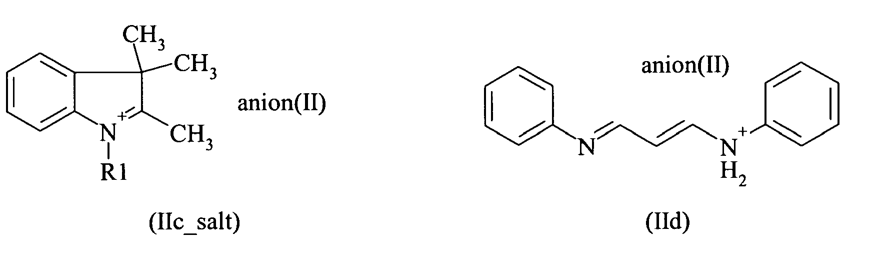

The condensation reaction is carried out in non-aqueous solvents and in mixtures thereof. Non-aqueous solvents are preferably selected from the group consisting of acetic acid or anhydride acetic; more preferably anhydride acetic is used. To neutralize the HCl of commercially available compound of formula (IId), one equivalent of sodium acetate was also added.

The condensation reaction is preferably done at a temperature of from 0°C to 200°C, more preferably of from 20°C to 150°C, even more preferably of from 30°C to 120°C.

Preferably, the condensation reaction is carried out under reflux at the reflux temperature of the solvent system used and at atmospheric pressure.

The condensation reaction time is preferably of from 10 min to 1 week.

Preferably the compound of formula (II_salt) is isolated following standard methods, in case of a precipitate preferably by filtration followed preferably by drying.

Another subject of the invention is the use of a compound of formula (IIc_salt) for the preparation of a compound of formula (II_salts).

Another subject of the invention is the use of a compound of formula (IId) for the preparation of a compound of formula (II_salts).

The compounds of formula (IIc_salt) are preferably prepared by alkylation reaction of the commercially available compound of formula (IIb), with the respective compound of formual (IIa) acting as an alkylating agent; the anion(II_cov) being selected from the group consisting halogen, preferably Cl, Br and I, even more preferably I; the R1 having the same meaning as described above, also with all its preferred embodiments.

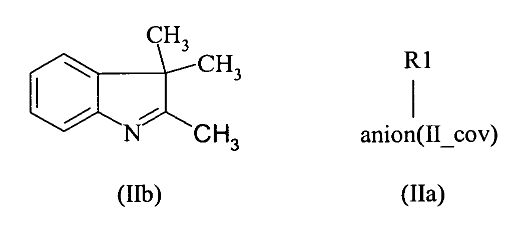

The alkylation reaction is carried out in non-aqueous solvents and in mixtures thereof. Non-aqueous solvents are preferably selected from the group consisting of aromatic solvents, alcohols, ketones or acetonitrile; more preferably from ketones or substituted benzenes, even more preferably ethylmethylketone is used.

The alkylation reaction is preferably carried out with excess of alkylating agent, more preferably the molar ratio of alkylating agent to compound of formula (IIb) is of from 5 to 1.

The alkylation reaction is preferably done at a temperature of from 0°C to 200°C, more preferably of from 20°C to 100°C, even more preferably of from 30°C to 90°C.

Preferably, the alkylation reaction is carried out under reflux at the reflux temperature of the solvent system used and at atmospheric pressure.

The alkylation reaction time is preferably of from 10 min to 1 week.

Preferably the compound of formula (IIc_salt) is isolated following standard methods, in case of a precipitate preferably by filtration followed preferably by drying.

Another subject of the invention is a compound of formula (II_salts), wherein the compound of formula (II) is compound of formula (1), and the anion(II) is selected from the group consisting of halides, preferably chloride, bromide and iodide, even more preferably iodide; especially a compound of formula (1_I).

### Preparation of compound of formula (20_salts)

The compound of formula (20_salts), especially the compound of formula (20_I), 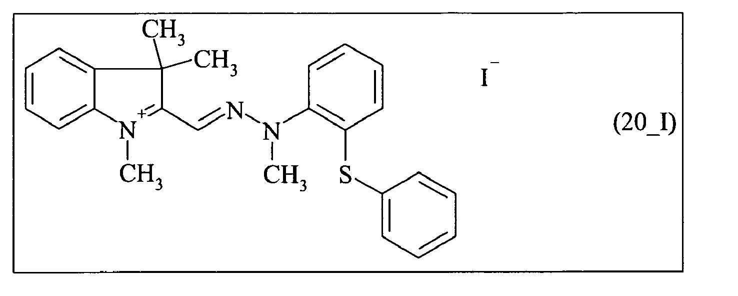 is prepared by an alkylation reaction of a compound of formula (20d) with a compound of formula (20_alk) acting as alkylating agent, the anion(II_cov) being selected from the group consisting halogen, preferably Cl, Br and I, even more preferably I.

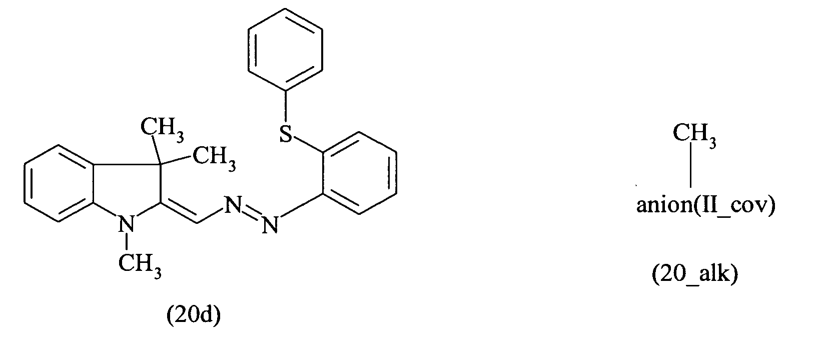

Preferably, the alkylation reaction is carried out in suspension or in solution, even more preferably in solution.

The alkylation reaction is preferably carried out in non-aqueous solvents and in mixtures thereof. Non-aqueous solvents are preferably selected from the group consisting of aromatic solvents, alcohols, ketones or acetonitrile; more preferably from ketones or substituted benzenes, even more preferably ethylmethylketone or chlorobenzene is used.

The alkylation reaction is preferably carried out with excess of alkylating agent, more preferably the molar ratio of alkylating agent to compound of formula (20d) is of from 5 to 1.

The alkylation reaction is preferably done at a temperature of from 0°C to 200°C, more preferably of from 20°C to 100°C, even more preferably of from 30°C to 90°C.

The alkylation reaction time is preferably of from 10 min to 1 week.

### The alkylation reaction is preferably done under atmospheric pressure

Preferably, the alkylation reaction is done under reflux and atmospheric pressure.

Preferably the compound of formula (20_salts) is isolated following standard methods, in case of a precipitate preferably by filtration followed preferably by drying.

The compound of formula (20d) is prepared by an azo coupling reaction of the compound of formula (20a), also called coupling agent, with a compound of formula (20b), also called diazo component; the compound of formula (20b) being preferably prepared by diazotization reaction of a compound of formula (20c), also called amine compound;

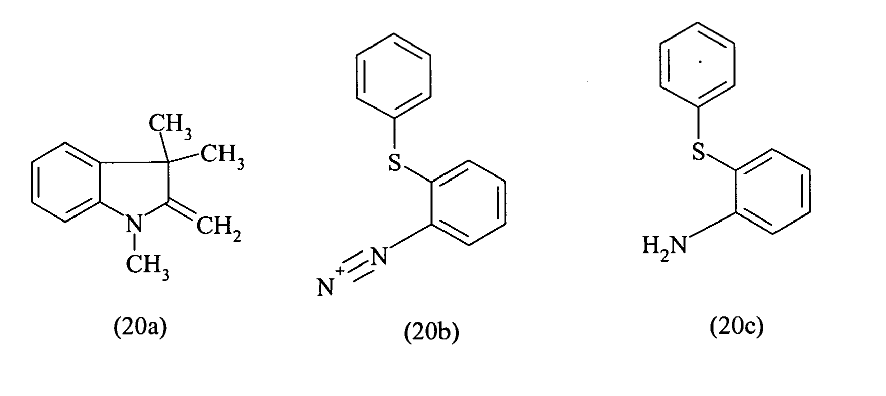

The diazo component has preferably chloride Cl- as counter ion, since the diazotization reaction of the amine compound preferably is done in aqueous hydrochloride acid.

The amine compound and the coupling agent are known and commercially available substances.

The azo coupling reaction is preferably carried out in suspension or in solution.

The azo coupling reaction is preferably carried out in water, non-aqueous solvents and in mixtures thereof. Non-aqueous solvents are preferably selected from the group consisting of alcohols, more preferably methanol, ethanol, propanol, butanol, pentanol, dipolar aprotic solvents, preferably dimethylformamide (DMF), DMSO, dimethylacetamide or N-methyl-pyrrolidinone (NMP) and pyridine, and water-immiscible solvents, preferably toluene or chlorobenzene. More preferably the azo coupling reaction is carried out in water, methanol or in mixture thereof.

The azo coupling reaction is preferably carried out with a stoichiometric ratio of coupling component and diazo component.

The azo coupling reaction is generally done at a temperature of from -30°C to 100°C, preference being given to temperatures of-10°C to 30°C, and particular preference to temperatures of -5°C to 30°C.

Preferably, the reaction time for the azo coupling reaction is preferably of from 30 min to 30 hours, more preferably of from 1 hour to 24 hours.

Preferably, the azo coupling reaction is done under atmospheric pressure.

The azo coupling reaction may be carried out in an acidic as well as an alkaline medium. Preference is given to pH < 10, particular preference to pH 3 to 9.

Preferably the azo ligand is isolated following standard methods, in case of a precipitate preferably by filtration followed preferably by drying.

A further subject of the invention is the use of a compound of formula (II_salts), also with all their preferred embodiments as defined above, for the preparation of compounds of formula (I).

### Optical Layer and Optical Data Recording Medium

A further subject of the invention is an optical layer, preferably for optical data recording, comprising at least one compound of formula (I), with the compound of formula (I) also in all its above described embodiments, particularly at least one compound of formula (10_1), (11_1), or (12_1), preferably comprising a composition C, with the composition C also in all its above described embodiments; and the use of said optical layer for optical data recording media. An optical layer according to the invention comprise preferably at least one, preferably two or three, more preferably three compounds of formula (I). A further subject of the invention therefore is an optical data recording medium comprising an optical layer comprising at least one compound of formula (I), preferably comprising a composition C.

Preferably, subject of the invention is a DVD-R optical data recording media, which comprises an optical layer comprising at least one compound of formula (I), preferably comprising a composition C.

Preferably, subject of the invention is a write only read many (WORM) type optical data recording medium capable of recording and reproducing information with radiation of a laser, preferably of a red laser at a wavelength from 630 to 670 nm, more preferably at around 650 nm, which comprises an optical layer comprising at least one compound of formula (I), preferably comprising a composition C.

Further, the invention relates to a method for producing an optical layer comprising the following steps
(a) providing a substrate,
(b) dissolving at least one compound of formula (I), particularly at least one compound of formula (10_1), (11_1), or (12_1), preferably dissolving a composition C, in an organic solvent to form a solution,
(c) coating the solution (b) on the substrate (a),
(d) evaporating the solvent to form an optical layer.

### (a) Substrate

The substrate, which functions as support for the layers applied thereto, is advantageously semi-transparent (transmittance T>10%) or preferably transparent (transmittance T>90%). The support can have a thickness of from 0.01 to 10 mm, preferably from 0.1 to 5 mm.

Suitable substrates are, for example, glass, minerals, ceramics or thermosetting or thermoplastic plastics. Preferred supports are glass and homo- or co-polymeric plastics. Suitable plastics are, for example, thermoplastic polycarbonates, polyamides, polyesters, polyacrylates and polymethacrylates, polyurethanes, polyolefins, polyvinyl chloride, polyvinylidene fluoride, polyimides, thermosetting polyesters and epoxy resins. The most preferred substrates are polycarbonate (PC) or polymethylmethacrylate (PMMA).

The substrate can be in pure form or may also comprise customary additives, for example UV absorbers as light-stabilizers for the optical layer.

The substrate is advantageously transparent over at least a portion of the range from 350 to 500 nm, so that it is permeable to at least 90% of the incident light of the writing or readout wavelength.

### (b) Organic solvents

Organic solvents are selected from the group consisting of C₁₋₈ alcohols, halogen substituted C₁₋₈ alcohols, C₁₋₈ ketones, C₁₋₈ ethers, halogen substituted C₁₋₄ alkanes, nitriles, preferably acetonitrile, and amides, and mixtures thereof.

Preferred C₁₋₈ alcohols or halogen substituted C₁₋₈ alcohols are for example methanol, ethanol, isopropanol, diacetone alcohol (DAA), 2,2,3,3-tetrafluoropropan-1-ol, trichloroethanol, 2-chloroethanol, octafluoropentanol or hexafluorobutanol, more preferred 2,2,3,3-tetrafluoropropan-1-ol.

Preferred C₁₋₈ ketones are for example acetone, methylisobutylketone, methylethylketone, or 3-hydroxy-3-methyl-2-butanone.

Preferred halogen substituted C₁₋₄ alkanes are for example chloroform, dichloromethane or 1-chlorobutane.

Preferred amides are for example DMF, dimethylacetamide or NMP.

### (c) Coating methods

Suitable coating methods are, for example, immersion, pouring, brush-coating, blade-application and spin-coating, as well as vapor-deposition methods carried out under a high vacuum. When pouring methods are used, solutions in organic solvents are generally used. When solvents are employed, care should be taken that the supports used are insensitive to those solvents. The optical layer is preferably applied by spin-coating with a dye solution.

### (d) Optical layer

The optical layer is preferably arranged between the transparent substrate and the reflecting layer. The thickness of the recording layer is from 10 to 1000 nm, preferably from 30 to 300 nm, more preferably from 70 to 250 nm, especially about 80 nm, for example from 60 to 120 nm.

The optical layer comprises a compound of formula (I), preferably it comprises a composition C, preferably in an amount sufficient to have a substantial influence on the refractive index, more preferably at least 30 % by weight, even more preferably at least 60 % by weight, especially at least 80 % by weight, the % by weight always based on the total weight of the optical layer.

Further customary components are stabilizers, for example ¹0₂-, triplet- or luminescence quenchers, melting-point reducers, decomposition accelerators or any other additives that have already been described in optical data recording media. Preferably, stabilizers or fluorescence-quenchers are added if desired.

Stabilizers, ¹0₂-, triplet- or luminescence-quenchers are, for example, metal complexes ofN- or S-containing enolates, phenolates, bisphenolates, thiolates or bisthiolates, hindered phenols and derivatives thereof such as o-hydroxyphenyl-triazoles or -triazines or other UV absorbers, such as hindered amines (TEMPO or HALS, as well as nitroxides or NOR-HALS), and also as cations diimmonium, ParaquatTM or Orthoquat salts, such as ®Kayasorb IRG 022, ®Kayasorb IRG 040, optionally also as radical ions, such as N,N,N',N'-tetrakis(4-dibutylaminophenyl)-p-phenylene amine-ammonium hexafluorophosphate, hexafluoroantimonate or perchlorate. The latter are available from Organica (Wolfen/DE); ®Kayasorb brands are available from Nippon Kayaku Co. Ltd.

In a preferred aspect, the present invention provides for an optical layer suitable for high-density recording material, e.g. of the WORM disc format, in a laser wavelength range of from 350-450nm, preferably around 405 nm.

### Preparation of the optical data recording medium

A method for producing an optical data recording medium comprising an optical layer according to the invention usually comprises the following additional steps
(e) applying a metal layer, also called reflective layer, onto the optical layer,
(f) applying a second polymer based layer to complete the disk, also called cover layer or protective layer.

### (e) Reflective layer

The application of the metallic reflective layer is preferably effected by sputtering, vapor-deposition in vacuum or by chemical vapor deposition (CVD). The sputtering technique is especially preferred for the application of the metallic reflective layer.

Reflecting materials suitable for the reflective layer include especially metals, which provide good reflection of the laser radiation, used for recording and playback, for example the metals of Main Groups III, IV and V and of the Sub-groups of the Periodic Table of the Elements. Al, In, Sn, Pb, Sb, Bi, Cu, Ag, Au, Zn, Cd, Hg, Sc, Y, La, Ti, Zr, Hf, V, Nb, Ta, Cr, Mo, W, Fe, Co, Ni, Ru, Rh, Pd, Os, Ir, Pt, Ce, Pr, Nd, Pm, Sm, Eu, Gd, Tb, Dy, Ho, Er, Tm, Yb and Lu and alloys thereof are especially suitable. Special preference is given to a reflective layer of aluminum, silver, copper, gold or an alloy thereof, on account of their high reflectivity and ease of production.

### (f) Cover layer

Materials suitable for the cover layer include plastics, which are applied in a thin layer to the support or the uppermost layer either directly or with the aid of adhesive layers. The material of the cover layer may for example be the same as the material of the substrate. It is advantageous to select mechanically and thermally stable plastics having good surface properties, which may be modified further.

The plastics may be thermosetting plastics and thermoplastic plastics. Preference is given to radiation-cured (e.g. using UV radiation) protective layers, which are particularly simple and economical to produce. A wide variety of radiation-curable materials are known. Examples of radiation-curable monomers and oligomers are acrylates and methacrylates of diols, triols and tetrols, polyimides of aromatic tetracarboxylic acids and aromatic diamines having C₁₋₄ alkyl groups in at least two ortho-positions of the amino groups, and oligomers with dialkylmaleinimidyl groups, e.g. dimethyl maleinimidyl groups.

A high-density optical data recording medium according to the invention therefore preferably is a recordable optical disc comprising: a first substrate, which is a transparent substrate with grooves, a optical layer (recording layer), which is formed on the first substrate surface using the compound of formula (I), preferably using a composition C, a reflective layer formed on the optical layer, a second substrate, which is a transparent substrate connected to the reflective layer with an attachment layer.

The optical data recording medium according to the invention is preferably a recordable optical disc of the WORM type. It may be used, for example, as a playable HD-DVD (high density digital versatile disc) or Blu-ray® disc, as storage medium for a computer or as an identification and security card or for the production of diffractive optical elements, for example holograms.

The optical data recording media according to the invention may also have additional layers, for example interference layers. It is also possible to construct optical data recording media having a plurality of (for example two) recording layers. The structure and the use of such materials are known to the person skilled in the art. Preferred, if present, are interference layers that are arranged between the recording layer and the reflecting layer and/or between the recording layer and the substrate and consist of a dielectric material of Ti0₂, Si₃N₄, ZnS or silicone resins.

These optical data recording media according to the invention can be produced by processes known in the art.

### Readout methods

The structure of the optical data recording medium according to the invention is governed primarily by the readout method; known function principles include the measurement of the change in the transmission or, preferably, in the reflection, but it is also known to measure, for example, the fluorescence instead of the transmission or reflection.

When the optical data recording medium is structured for a change in reflection, the following structures can be used: transparent support / recording layer (optionally multilayered) / reflective layer and, if expedient, protective layer (not necessarily transparent); or support (not necessarily transparent) / reflective layer / recording layer and, if expedient, transparent protective layer. In the first case, the light is incident from the support side, whereas in the latter case the radiation is incident from the recording layer side or, where applicable, from the protective layer side. In both cases the light detector is located on the same side as the light source. The first-mentioned structure of the recording material to be used according to the invention is generally preferred.

When the optical data recording medium is structured for a change in light transmission, the following different structure comes into consideration: transparent support/recording layer (optionally multilayered) and, if expedient, transparent protective layer. The light for recording and for readout can be incident either from the support side or from the recording layer side or, where applicable, from the protective layer side, the light detector in this case always being located on the opposite side.

Suitable lasers are those having a wavelength of 330-500 nm, for example commercially available lasers having a wavelength of 405 to 414 nm, especially semi-conductor lasers. The recording is done, for example, point for point, by modulating the laser in accordance with the mark lengths and focusing its radiation onto the recording layer. It is known from the specialist literature that other methods are currently being developed which may also be suitable for use.

The process according to the invention allows the storage of information with great reliability and stability, distinguished by very good mechanical and thermal stability and by high light stability and by sharp boundary zones of the pits. Special advantages include the high contrast, the low jitter and the surprisingly high signal/noise ratio, so that excellent readout is achieved.

The readout of information is carried out according to methods known in the art by registering the change in absorption or reflection using laser radiation.

The invention accordingly relates also to a method for the optical data recording, storage and playback of information, wherein an optical data recording medium according to the invention is used. The recording and the playback advantageously take place in a wavelength range of from 330 to 500 nm.

The compounds of formula (I), preferably the compositions C, provide for particularly preferable properties when used in optical layers for optical data recording media according to the invention. They possess the required optical characteristics, demonstrated when used in the form of a solid film:
- an advantageously homogeneous, amorphous and low-scattering optical layer,
- a high refractive index at the longer wavelength flank of the absorption band, which preferably achieves n values of the refractive index of from 1.0 to 3.0 in the range of from 330 to 500 nm,
- a high sensitivity under laser radiation of high power density and good playback characteristics in the desired spectral range,
- an enhanced photosensitivity and stability (in daylight and under laser radiation of low power density) compared to dyes already known in the art,
- an uniform script width and a high contrast,
- an absorption maximum (lambda-max) in the preferred range between 330 nm and 500 nm as being preferred for blue laser applications, more precisely from 380 to 460 nm,
- a decomposition point (DP) in the preferred temperature range between 180°C and 300°C, more precisely 200°C to 290°C
- a sufficient heat release (HR)

Recording performance of a compound is related to specific parameters measured on disc like:
- a low simulated bit error rate (SbER)
- a low inner parity error rate (PI error)
- a high reflectivity (R)
- a low laser recording power (Pw: power, or OPC: optimum power control): the lower the better
- good readout stability at different laser reading powers
- an appropriate partial response signal to noise ratio (PRSNR): the higher the better

The absorption edge is surprisingly steep even in the solid phase.

The compounds of formula (I), preferably the compositions C, also show a narrow decomposition temperature of 180 to 350°C, fitting with the thermal requirements. Additionally, these compounds show a high solubility in organic solvents, which is ideal for the spin-coating process to manufacture optical layers.

The use of a compound of formula (I), preferably the use of a composition C, in an optical layer for optical data recording allows unexpectedly data recoding at higher speeds than the conventional 1X speed in HD-DVD and Blu-ray-discs.

As a result of the use of the dyes of the invention, the recording media of the invention advantageously have homogeneous, amorphous and low scattering recording layers. Further advantages is the light stability in day light and under laser radiation of 0.4 mW, combined with a high sensitivity under laser radiation of moderate, this means as low as possible, power density (OPC preferably less than 8.0 mW for 1X speed and preferably less than 11 mW for 2X speed), the good thermal and storage stability. Especially in case of recording at higher speed, the OPC required should be as low as possible.

### EXAMPLES

### UV-vis

For UV-vis spectra, λ max and ε values of a compound are determined by using an UV-vis spectrophotometer, the compound was dissolved in CH₂Cl₂, DMSO or in tfp. The values are obtained by balancing the measurements performed on compound solutions at three different concentrations.

### Melting point MP)

For the determination of melting point, the compound or the composition is incorporated in a glass capillary. The capillary was heated using the following profile: temperature range from 20 to 350 °C, heating rate 2 °C/min.

### Thermal Decomposition: Decomposition point (DP) and heat release (HR)

For the determination of DP and HR, the compound is incorporated into a sealed aluminum pan. Analysis conditions are as following: Temperature range from 25 to 400°C, heating rate 10°C/min, nitrogen flow of 50 ml/min. Values are determined by single measurement. Additionally, thermal decomposition is also being observed while measuring the melting point.

### Partial response signal to noise ratio (PRSNR)

A definition and the measuring techniques of PRSNR are described in a book available from DVD Format Logo Licensing Co., Ltd. for example, Annex H of Version 0.9, PART 1 Physical Specifications, DVD Specifications for High Density Read-Only Disk. The higher the PRSNR the better.

### Simulated bit error rate (SbER)

A definition and the measuring techniques of SbER are described in a book available from DVD Format Logo Licensing Co., Ltd. for example, Annex H of Version 0.9, PART 1 Physical Specifications, DVD Specifications for High Density Read-Only Disk. The lower the SbER the better.

PRSNR and SbER are measured in a state in which information has been recorded in the adjacent tracks.

### Reflectivity (R)

A definition and the measuring techniques for the light reflectivity (R) is described in a book available from DVD Format Logo Licensing Co., Ltd. for example, Annex D of Version 0.9, PART 1 Physical Specifications, DVD Specifications for High Density Read-Only Disk. The higher the R the better.

### Cycle number

The degree of degradation of various parameters, e.g. of the PRSNR and SbER, due to repetitive read out is measured. The higher the cycle number until reaching the minimum specifications or a comparable performance the better.

"Ex." means example, "Comp. Ex." means comparative example.

### Example 1

107.2 g of 2,3,3-trimethylindolenine and 183.9 g of butyliodide were refluxed overnight in 160 ml of ethylmethylketone. The temperature was cooled down to 40°C and 120 ml of ethyl acetate were added dropwise. Temperature was allowed to reach room temperature and 100 ml of ethyl acetate were again added. The resulting precipitate was filtered and washed with 200 ml of ethyl acetate. The obtained solid was stirred in 400 ml of ethyl acetate and filtered again. The white solid was washed with 1 L of hexane and dried for 24 h at 60°C under vacuum to afford 190.7 g of compound of formula (1c_I). 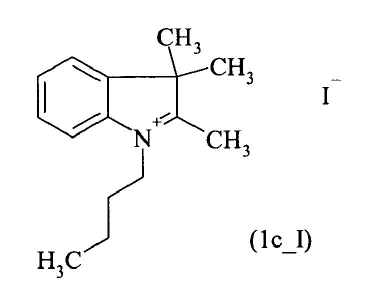 20.6 g of compound of formula (1c_I) are added to 50 ml of acetic anhydride followed by addition of 8.0 g of compound of formula (IId). The obtained yellowish suspension is then heated to 100°C until a dark-green solution is observed. 2.5 g of sodium acetate are added to the reaction mixture which is then heated to 100°C for 16 hours. The resulting mixture is stirred for further 16 hours at 25°C. Temperature was cooled down to 0°C, the precipitate was filtered, washed with 100 ml of cold water and dried under vacuum at 60°C for 24 hours to afford 10.5 g of the compound of formula (1_I).

### Example 2

87 g of conc. aqueous HCl were added dropwise to a solution composed of 54.7 g of compound of formula (20c) in 600 ml of methanol and 100 ml of water. Temperature was decreased to 0°C with an ice bath and 67.7 ml of a solution of aqueous sodium nitrite (33.3% by weight) were added dropwise while temperature was maintained below 5°C. The resulting solution was stirred at 0°C for 1.5 hour. 2 ml of a 10% aqueous solution of amidosulfonic acid 10% were added and the mixture was pumped into a mixture composed of 46.9 g of compound of formula (20a), 85.9 g of Na₂CO₃ and 400 ml of methanol. After complete addition, the mixture is stirred for a further 1 hour at room temperature. The brown precipitate was filtered, washed with 8000 ml of water, dried under vacuum at 60°C for 24 hours to afford 85.9 g of compound of formula (20d) as a brown solid.

The 85.4 g of compound of formula (20d) were suspended in 150 ml of methylethylketone, 95.3 g of methyliodide were added and the resulting mixture was refluxed for 48 hours. Temperature was cooled to room temperature and the formed precipitate was filtered, washed with 150 ml of methylethylketone and dried under vacuum at 60°C for 24 hours. 63.2 g of compound of formula (20_I) were obtained as an orange solid.

Table (A4) shows the phys-chem properties of the compound of formula (1_I) and compound of formula (20_I).

| **Table (A4)** | | | | |
|---|---|---|---|---|
| **Ex.** | **Compound of formula** | λ **max [nm]** | ε **(at** λ **max) [L / g * cm]** | **MP/ DP [°C]** |
| **1** | (1_I) | 655 | 282 | 159 (MP) |
| **2** | (20_I) | 403 | 60 | 207 (MP), 247 (DP) |

### Example 3a

34.65 g of ethyl cyanoacetate were added dropwise to 17.48 g of allylamine. The resulting mixture was refluxed for 1hour. Temperature was cooled down to 70°C and 43.38 g of ethyl acetoacetate were added dropwise followed by 48 g of piperidine and 166 ml of toluene respectively. The resulting mixture was refluxed overnight. Toluene was distilled off, 160 ml of water were added and the mixture was allowed to cool to room temperature. The resulting slurry was transferred dropwise to 150 ml of cold methanol (ice bath). During the transfer, the pH is maintained to pH 1 by addition of concentrated aqueous HCl. The resulting yellowish precipitate was filtered, washed 5 times with each 200 ml of water and dried under vaccum at 60°C for 24 hours. 37.7 g of compound of formula (c1) were obtained as a white solid.

8.68 g of 4-nitro-2-aminophenol were added to 100 ml of water followed by dropwise addition of 16.1 g of concentrated aqueous HCl. Temperature was cooled to 0°C and 10.4 ml of a aqueous solution of sodium nitrite (33.3% by weight) were added dropwise, keeping the temperature below 5°C. The yellow mixture was stirred at this temperature for 1 hour. The mixture was then pumped in a mixture containing 9.51 g of compound of formula (c1) and 12.4 g of sodium acetate in 200 ml of water. After complete addition, the resulting mixture was stirred 1hour at room temperature. The resulting orange precipitate was filtered, washed 6 times of each 250 ml of water and dried under vacuum at 60°C for 24 hours. 16.12 g of compound of formula (d1) were obtained as a yellow solid.

7.1 g of compound of formula (d1), 2.81 g of CoSO₄·7H₂O and 100 ml of acetonitrile were refluxed for 20 minutes. 6.13 g of triethylamine were added dropwise and the resulting mixture was refluxed under aerobic conditions for 1h 30 min. After being cooled to room temperature, the solution was filtered and the filtrate was distilled. To the resulting purple slurry were added dropwise 100 ml of ethanol and the mixture was refluxed for 1hour. After being cooled to room temperature, the greenish-brown precipitate was filtered, washed with 50 ml of ethanol, 300 ml of water and dried under vaccum at 60°C for 24 hours. 8.06 g of compound of formula (10_6) were obtained as a greenish-brown solid.

### Example 3b

5.14 g of compound of formula (1_I) dissolved in 100 ml of ethanol were filtered before being added dropwise to a refluxing mixture of 5 g of compound of formula (10_6) in 50 ml of acetonitrile. After complete addition, reflux was continued for four hours. The temperature was then decreased to 5°C and the formed precipitate was filtered and washed with ethanol (50 ml). The solid was dissolved in 250 ml of dichloromethane and filtered through a short pad of celite. To the filtrate was added 250 ml of ethanol and dichloromethane was slowly evaporated. The precipitate was filtered, washed respectively with 100 ml of ethanol and 100 ml of water and finally dried for 24 hours at 60°C under vacuum to afford 4 g of compound of formula (10_1) as a green solid.

### Example 4a

50g of propargylamine was added in 20 minutes at room temperature to 51.36g of cyano acetic acid ethylester under nitrogen. The resulting mixture was stirred 12 hours at room temperature under nitrogen. The precipitate was filtered, washed with toluene (75 ml) and dried in vaccum at 80°C to afford 48.57 g of 2-Cyano-*N*-prop-2-inyl-acetamide.

51.79g of acetyl acetic acid ethylester were dissolved in ethanol (160ml) at room temperature under nitrogen. The solution was cooled to 0°C and 27.08g of sodium ethylate were added. The resulting mixture was stirred for 10 min. and 48.57g of 2-cyano-N-prop-2-inyl-acetamide were added portionwise. The suspension was refluxed for 15 hours while ethanol was exchanged three times to remove the water formed during the reaction. After being cooled to room temperature, the mixture was dilute with ethanol, the solid was filtered and dried in vacuum at 80 °C to afford compound of formula (c2).

The preparation of compound of formula (d2) from compound of formula (c2) according to the example 3a was done using 21.9 g of compound of formula (c2) with regard to 21.5 g of the respective amine compound to yield 36 g of compound of formula (d2). The amine compound for diazotization was 2-amino-4-nitro-phenol.

The preparation of compound of formula (11_6) from compound of formula (d2) according to example 3a was done using 17.1 g of compound of formula (d2) with regard to 6.1 g of CoSO₄·7H₂O to yield 15.3 g of compounds of formula (11_6).

### Example 4b

Compound of formula (11_6) (21g), compound of formula (1_I) (30g) and triethanolamine (11g) were refluxed 24 hours in ethanol (800ml). Temperature was allowed to cool to room temperature and the precipitate was filtered and washed with ethanol (3×50ml). The greenish-brown solid was dissolved in dichloromethane (800ml) and filtered through a pad of celite. The green filtrate was concentrated under reduce pressure until a volume of 200 ml. 200 ml of ethanol were added and dichloromethane was removed under reduce pressure. The precipitate was filtered, washed with ethanol (3×40ml) and finally dried 24 hours at 60°C under vacuum to afford 9.5g of the desired compound.

### Example 5a

23.6 g of 2-amino-4-nitro-6-acetamido-phenol were added to 190 ml of water followed by dropwise addition of 36.0 g of concentrated aqueous HCl. Temperature was cooled to 0°C and 25.5 ml of a aqueous solution of sodium nitrite (33.3% by weight) were added dropwise, keeping the temperature below 5°C. The yellow mixture was stirred at this temperature for 1 hour. The mixture was then pumped in a mixture containing 23.1 g of compound of formula (c3) and 45.9 g of sodium acetate in 210 ml of water. After complete addition, the resulting mixture was stirred 1hour at room temperature. The resulting orange precipitate was filtered, washed 8 times of each 100 ml of water and dried under vacuum at 60°C for 24 hours. 37.5 g of compound of formula (d3) were obtained as a yellow solid.

22.9 g of compound of formula (d3), 15.0 g of CoSO₄·7H₂O and 1000 ml of acetonitrile were refluxed for 20 minutes. 16.3 g of triethylamine were added dropwise and the resulting mixture was refluxed under aerobic conditions for 1h 30 min. After being cooled to room temperature, the solution was filtered and the filtrate was distilled. To the resulting purple slurry were added dropwise 160 ml of ethanol and the mixture was refluxed for 1hour. After being cooled to room temperature, the greenish-brown precipitate was filtered, washed with 60 ml of ethanol, 180 ml of water and dried under vaccum at 60°C for 24 hours. 24.2 g of compound of formula (12_6) were obtained as a greenish-brown solid.

### Example 5b

Compound of formula (12_6) (11.14 g) and compound of formula (1_I) (6.53 g) were refluxed for 5 hours in ethanol (160ml). After being cooled to 0°C (ice-bath), the precipitate was filtered, successively washed with ethanol (2×10 ml), water (2×10 ml) and finally dried for 24 hours at 60°C under vacuum to afford 13.4 g of the desired compound. This latter was then dispersed in 600 ml of dichloromethane and refluxed for 2 hours. The suspension is then filtered while hot on a pad of celite. The dark filtrate is concentrated under reduced pressure to dryness and dried at 60°C under vacuum to afford 10.4 g of the desired compound.

### Example 6

9.4 g of compound of formula (20_I) were refluxed for 1hour in 150 ml of ethanol. Temperature was decreased to 60°C and the solution was added dropwise to a refluxing mixture composed of 10.4 g of compound of formula (10_6) and 100 ml of acetonitrile. After complete addition, the mixture was refluxed for 4h. After being cooled to room temperature, the precipitate was filtered, washed with 30 ml of ethanol, 1000 ml of water and dried under vaccum at 60°C for 24 hours. 10.7 g of compound of formula (10_20) were obtained as a brown solid.

**Table (A6) shows the phys-chem properties of the compounds of formulae (I) and compound of formula (10_20).**

| **Table (A6)** | | | | |
|---|---|---|---|---|
| **Ex.** | **Compound of formula (I)** | λ **max [nm]** | ε **(at** λ **max) [L / g * cm]** | **MP/ DP [°C]** |
| **3b** | (10_1) | 655 | 221 | 277 (DP) |
| **4b** | (11_1) | 655 | 178 | 265 (DP) |
| **5b** | (12_1) | 655 | 178 | 270 (DP) |
| **6** | (10_20) | 483 | 64 | 285 (DP) |

## Claims

1. A compound of formula (I), 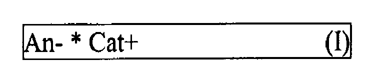 Cat+ being a compound of formula (II); 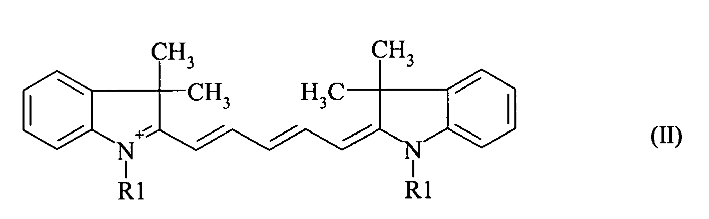 An- being a compound of formula (III); 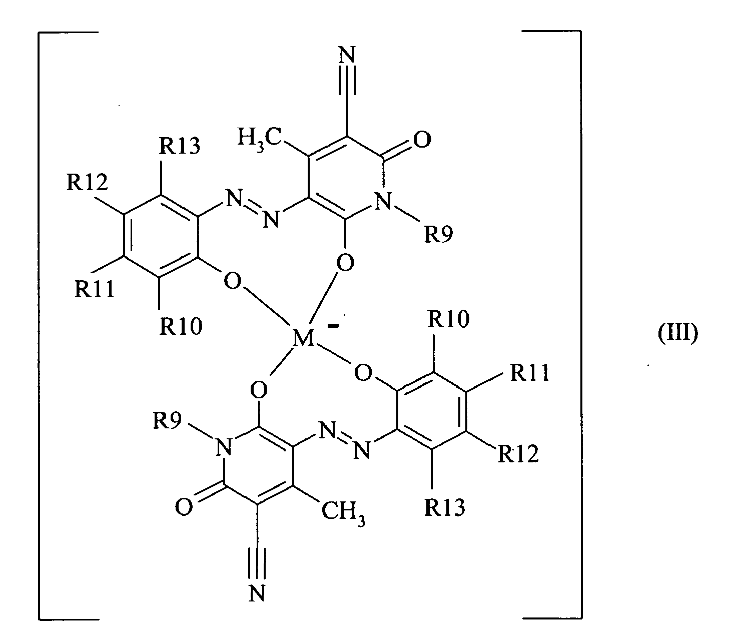
M represents a trivalent metal atom, preferably selected from groups 3, 4 5, 6, 7, 8, 9, 10, 11 and 12 of the Periodic Table of the Chemical Elements;
R9 is n-butyl, allyl or propargyl;
R1 represents C₁₋₁₀ alkyl;
R10, R11, R12 and R13 are identical or different and independently from each other selected from the group consisting of H, CN, CF₃, halogen, NO₂, OH, SH, SO₂-NR²¹R²², CO-R²⁰, SO₂R²⁰, CO-NR²¹R²², C₁₋₁₀ alkyl, C₃₋₁₀ cycloalkyl, the C₁₋₁₀ alkyl and the C₃₋₁₀ cycloalkyl being independently from each other unsubstituted or substituted by 1 to 4 identical or different substituents, the substituents being independently from each other selected from the group consisting of C₁₋₁₀ alkyl, halogen, OH, CN, CF₃, C₆₋₁₂ aryl and NR²¹R²², C₆-C₁₂ aryl, O-C₆₋₁₂ aryl, S-C₆₋₁₂ aryl, the C₆₋₁₂ aryl and the O-C₆₋₁₂ aryl and the S- C₆₋₁₂ aryl being unsubstituted or substituted by 1 to 4 identical or different substituents, the substituents being independently from each other selected from the group consisting of C₁₋₁₀ alkyl, C₃₋₁₀ cycloalkyl, OH, NO₂, CN, halogen, CF₃, C₆₋₁₂ aryl, O-C₁₋₁₀ alkyl, S-C₁₋₁₀ alkyl and NR²¹R²², O-C₁₋₁₀ alkyl, S-C₁₋₁₀ alkyl, O-C₃₋₁₀ cycloalkyl, S-C₃₋₁₀ cycloalkyl, NHCOR²⁰ and NR²¹R²²;
with the proviso, that if R9 is n-butyl, then R10 is -NHCOCH₃;
the R²¹ and R²² residues are identical or different and independently from each other selected from the group consisting of H, C₁₋₁₀ alkyl, C₆₋₁₂ aryl and C₁₋₁₂ alkyl-NR²³R²⁴;
the R²³ and R²⁴ residues are identical or different and independently from each other selected from the group consisting of H, C₁₋₁₀ alkyl and C₆₋₁₂ aryl;
the R²⁰ residues are identical or different and independently from each other selected from the group consisting of OH, C₁₋₆ alkyl, C₆₋₁₀ aryl and O-C₁₋₆ alkyl.

2. A compound of formula (I) according to claim 1,
M is selected from the group consisting of Co, Cr, Fe and Al;
R9 is n-butyl, allyl or propargyl;
R1 represents C₁₋₄ alkyl;
R10 is H or -NHCOCH₃;
with the proviso, that if R9 is n-butyl, then R10 is -NHCOCH₃;
R11 is H or NO₂;
R12 is NO₂;
R13 is H.

3. A compound of formula (I) according to claim 1 or 2,
M is selected from the group consisting of Co, Fe and Al;
R9 is n-butyl, allyl or propargyl;
R1 represents n-butyl;
R10 is H or -NHCOCH₃;
with the proviso, that if R9 is n-butyl, then R10 is -NHCOCH₃;
R12 is NO₂;
R11 and R13 are H.

4. A compound of formula (I) according to claim 1, wherein
Cat+ is a compound of formula (1); 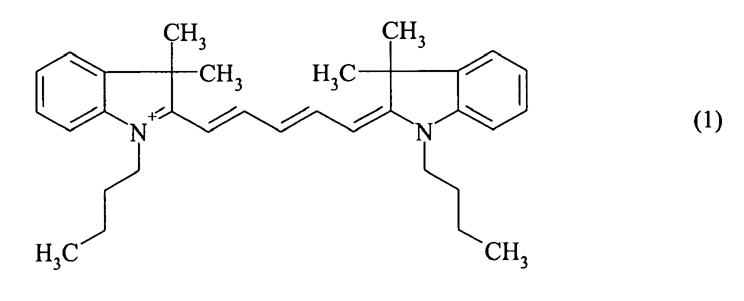 and
An- is a compound of formula (10), (11) or (12).

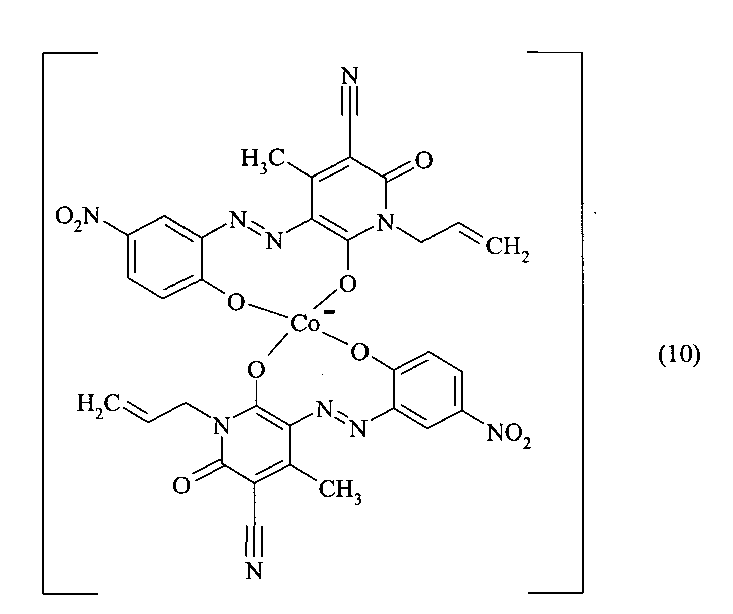

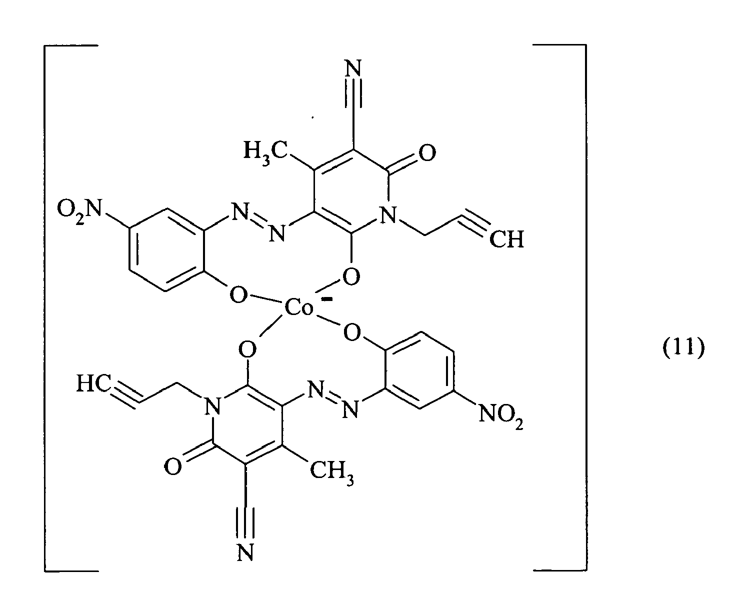

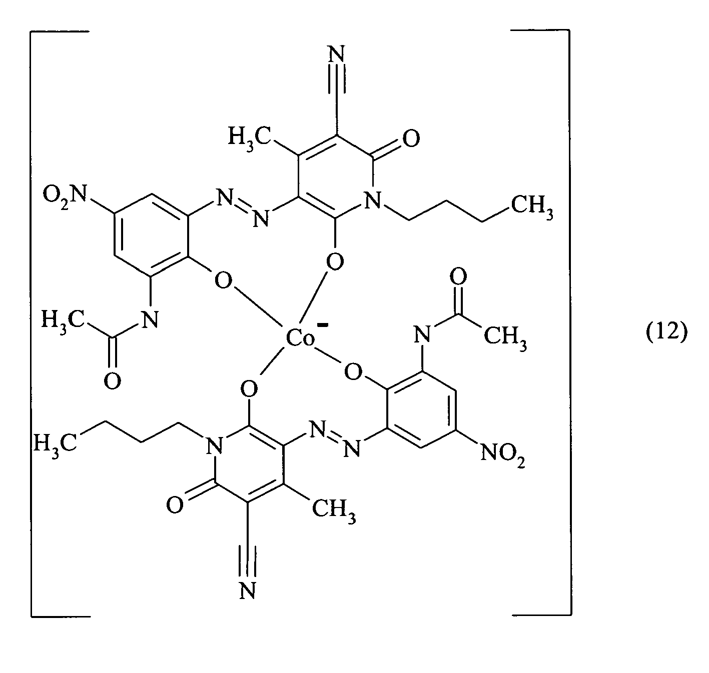

5. A composition C comprising a component A and a component B,
the component A being a compound of formula (I) as defined in one or more of claims 1 to 4, and
the component B being a compound of formula (10_20); 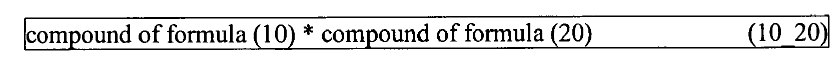 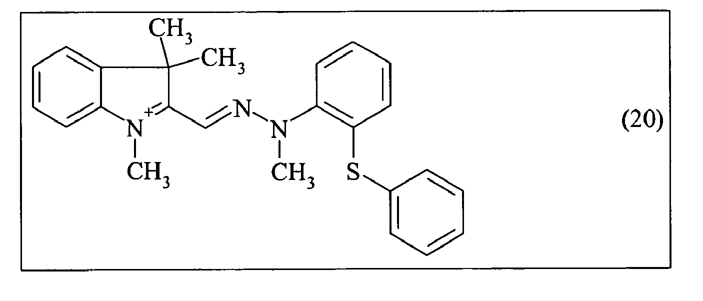 with the compound of formula (10) being as defined in claim 4.

6. Use of a compound of formula (I), as defined in one or more of claims 1 to 4, or of a composition C, as defined in claim 5, in an optical layer for optical data recording.

7. Use according to claim 6 of a compound of formula (I), as defined in one or more of claims 1 to 4, or of a composition C, as defined in claim 5, as a dye in an optical layer for optical data recording.

8. Use of a compound of formula (10_20) as defined in claim 5, for the preparation of a composition C as defined in claim 5.

9. Use of a compound of formula (I) as defined in one or more of claims 1 to 4, for the preparation of a composition C as defined in claim 5.

10. Use of a compound of formula (III_6), 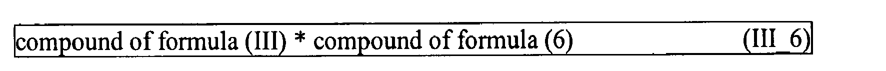 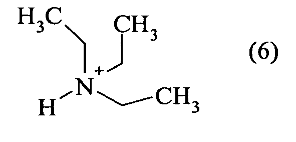 the compound of formula (III) being as defined in one or more of claims 1 to 4,
for the preparation of a compound of formula (I) as defined in one or more of claims 1 to 4.

11. Use of a compound of formula (II_salts), 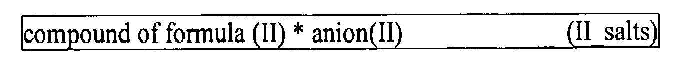 the compound of formula (II) being as defined in one or more of claims 1 to 4,
the anion(II) being selected from the group consisting of halides,
for the preparation of a compound of formula (I) as defined in one or more of claims 1 to 4.

12. A compound of formula (II_salts) as defined in claim 11.

13. A compound of formula (1_I).

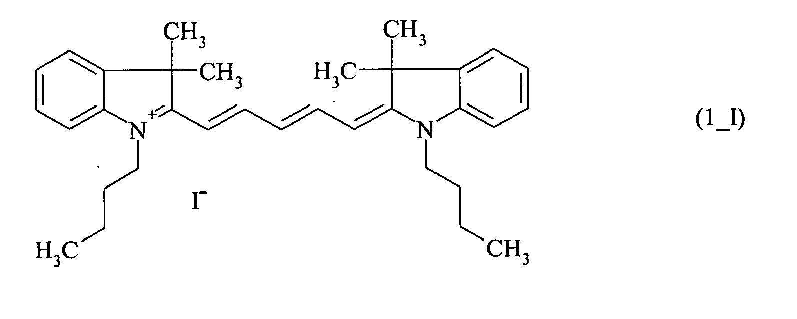

14. Use of a compound of formula (IIc_salt), 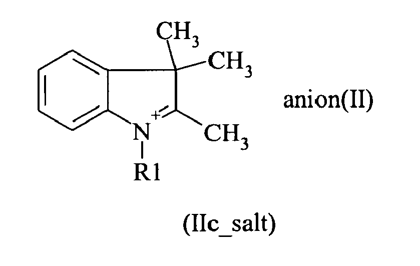 with R1 begin as defined in anyone of claims 1 to 3, and
with anion(II) being as defined in claim 11,
for the preparation of a compound of formula (II_salts) as defined in claim 12.

15. Use of a compound of formula (IId), 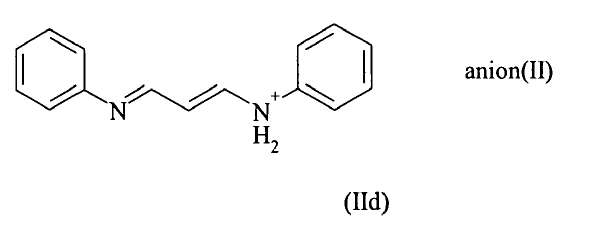 with anion(II) being as defined in claim 11,
for the preparation of a compound of formula (II_salts) as defined in claim 12.

16. An optical layer comprising a compound of formula (I) as defined in one or more of claim 1 to 4, or comprising a composition C as defined in claim 5.

17. A method for producing an optical layer as defined in claim 16, comprising the following steps
(a) providing a substrate,
(b) dissolving at least one compound of formula (I) as defined in one or more of claims 1 to 4, or dissolving a composition C as defined in claim 5, in an organic solvent to form a solution,
(c) coating the solution (b) on the substrate (a),
(d) evaporating the solvent to form an optical layer.

18. An optical data recording medium comprising an optical layer as defined in claim 16.
